# EUROPÄISCHE PATENTSCHRIFT

(11) **EP 0 962 434 B1**
(45) Veröffentlichungstag und Bekanntmachung des Hinweises auf die Patenterteilung: **27.11.2002**
(21) Anmeldenummer: 99110068.6
(22) Anmeldetag: 22.05.1999
(51) Int. Cl.: C07B 37/04, C07C 15/12, C07C 22/08, C07C 43/215, C07C 43/23, C07C 49/796, C07C 205/56, C07C 233/11

(54) **Verfahren zur Herstellung von aromatischen Olefinen unter Katalyse von Palladiumkatalysatoren mit Phosphitliganden**
Process for the preparation of aromatic olefins using palladium catalysts containing phosphite ligands
Procédé pour la préparation d'oléfines aromatiques utilisant des catalyseurs de palladium contenant des ligands phosphites

(30) Priorität: 06.06.1998 DE 19825454
(43) Veröffentlichungstag der Anmeldung: 08.12.1999
(73) Patentinhaber: Degussa AG, 40474 Düsseldorf (DE)
(72) Erfinder: Beller, Matthias Prof. Dr., 18119 Rostock (DE); Zapf, Alexander, 83024 Rosenheim (DE); Riermeier, Thomas, 65931 Frankfurt (DE)

(56) Entgegenhaltungen:
- L. HONG: "Preparation of polymer-supported catalysts containing phosphorus palladium complexes and quaternary onium groups, and their application to the vinylation reaction" REACTIVE POLYMERS, Bd. 16, Nr. 2, 1992, Seiten 181-197, XP002112144 Amsterdam
- M. BELLER: "Phosphites as ligands for efficient catalysis of Heck reactions" SYNLETT, Nr. 7, Juli 1998 (1998-07), Seiten 792-793, XP002112145 STUTTGART DE

## Beschreibung

Die vorliegende Erfindung betrifft ein Verfahren zur Herstellung von aromatischen Olefinen mit Katalysatoren auf Basis von Palladium-Katalysatoren mit Phosphitliganden.

Olefine, insbesondere aromatische Olefine wie Zimtsäurederivate, Styrole bzw. Stilbene haben technische Bedeutung als Feinchemikalien, Ausgangsprodukte für Polymere, UV-Absorber, Agrochemikalien und Wirkstoffvorprodukte.

Eine häufig angewandte Methode zur Synthese von aromatischen Olefinen im universitären Bereich ist die Heck-Reaktion, bei der lod- oder Bromaromaten und in Ausnahmefällen Chloraromaten mit Olefinen in Gegenwart von Palladiumkatalysatoren umgesetzt werden. Übersichtsartikel, die diese Methodik beschreiben, findet man beispielsweise in R.F. Heck, Org. React. 1982, 27, 345 und in B. Cornils, W. A. Herrmann, Applied homogeneous catalysis with organometallic compounds, Vol. 2, p. 712, VCH, Weinheim 1996.

Katalysatoren, die im Rahmen der Heck-Reaktion verwendet werden, sind Palladiumverbindungen. Obwohl sowohl Palladium (II)-, als auch Palladium (0)-Komplexe bei Heck-Reaktionen eingesetzt werden, ist es doch allgemein akzeptiert, daß Palladium (0)-Verbindungen die eigentlichen Katalysatoren der Reaktion sind. Insbesondere formuliert man gemäß Angaben in der Literatur koordinativ ungesättigte 14- und 16-Elektronen Palladium (0)-Spezies, welche mit Donorliganden wie Phosphanen stabilisiert werden. Beim Einsatz von Aryliodiden als Edukten in Heck-Reaktionen ist es möglich, auch auf Phosphanliganden zu verzichten. Allerdings sind Aryliodide sehr teure Ausgangsverbindungen, die darüberhinaus stöchiometrische Mengen an lodsalz-Abfällen produzieren. Kostengünstigere Edukte für die Heck-Reaktion wie Arylbromide oder Arylchloride benötigen den Zusatz von stabilisierenden Liganden, um katalytisch aktiviert und umgesetzt zu werden.

Die für Heck-Reaktionen beschriebenen Katalysatorsysteme weisen häufig nur mit nicht ökonomischen Ausgangsmaterialien wie lodaromaten und aktivierten Bromaromaten befriedigende katalytische Wechselzahlen ("turnover numbers" = TON) auf. Ansonsten müssen bei deaktivierten Bromaromaten und insbesondere bei Chloraromaten generell große Mengen an Katalysator - üblicherweise 1 bis 5 mol% - zugesetzt werden, um technisch nutzbare Umsätze zu erzielen. Aufgrund der Komplexität der Reaktionsgemische ist zudem kein einfaches Katalysatorrecycling möglich, so daß auch die Katalysatorkosten in der Regel einer technischen Realisierung entgegenstehen. Neuere Katalysatorsysteme basierend auf cyclometallierten Phosphanen ergeben zwar für eine Reihe von Bromaromaten befriedigende Katalysatoraktivitäten, jedoch müssen die Katalysatoren aus teuren alkylierten Phosphanen separat hergestellt werden. Darüberhinaus sind Chloraromaten auch mit diesen Katalysatoren noch nicht technisch befriedigend zu aktivieren (W. A. Herrmann, C. Broßmer, K. Öfele, T. Priermeier, M. Beller, H. Fischer, *Angew. Chem.* **1995**, *107*, 1989, Strukturell definierte Katalysatoren für die Heck-Olefinierung von Chlor- und Bromaromaten). Wenn hohe Ausbeuten erzielt wurden, mußten sehr hohe und damit sehr teure Katalysatormengen eingesetzt werden, da die Katalysatorproduktivität TON auch mit diesen Systemen bisher gering ist.

Aus den genannten Gründen bestand ein großer Bedarf nach einem neuen Palladium-Katalysatorsystem, das kostengünstige Liganden aufweist und das nicht die Nachteile der bekannten katalytischen Verfahren zeigt, das für die großtechnische Durchführung geeignet ist und das Olefine in hoher Ausbeute, Katalysatorproduktivität und Reinheit liefert.

Diese Aufgabe wird gelöst durch ein Verfahren zur Herstellung von mono-, bioder/und polyfunktionellen Olefinen der Formeln (la), (Ib), (Ic) oder/und (Id), 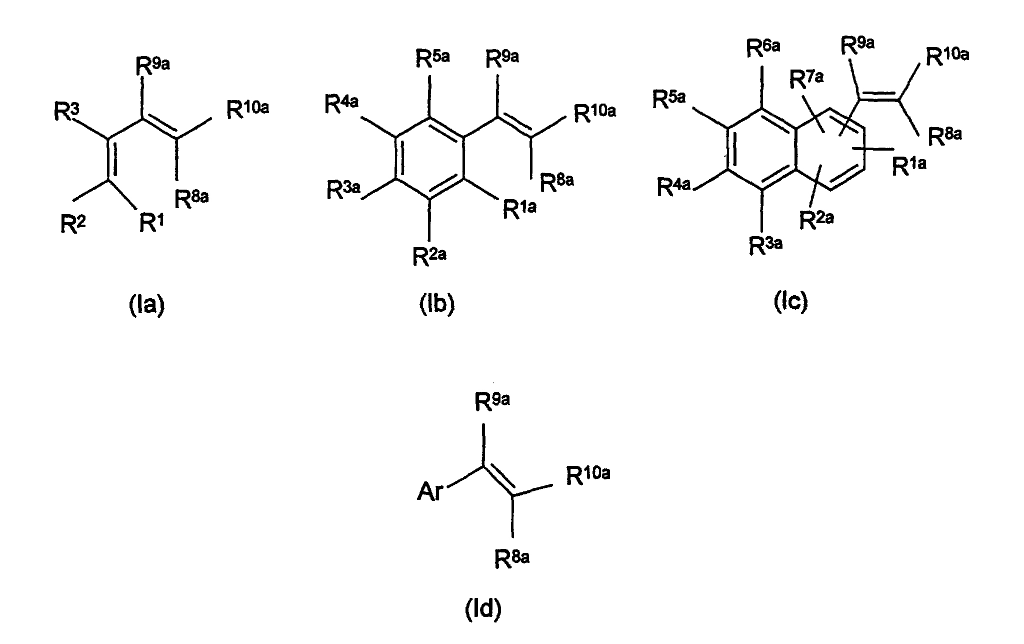 worin R¹ bis R³ unabhängig voneinander Wasserstoff, (C₁-C₈)-Alkyl, CN, COOH, COO-Alkyl-(C₁-C₈), CO-Alkyl-(C₁-C₈), Aryl-(C₆-C₁₀), COO-Aryl-(C₆-C₁₀), CO-Aryl-(C₆-C₁₀),
O-Alkyl-(C₁-C₈), O-CO-Alkyl-(C₁-C₈), N-Alkyl₂-(C₁-C₈) bedeutet und Aryl für einen bis zu 10C-Atome enthaltenden aromatischen Rest steht,
wobei dieser Rest bis zu fünf Substituenten tragen kann, die unabhängig voneinander Wasserstoff, (C₁-C₈)-Alkyl, O-Alkyl-(C₁-C₈), OCO-Alkyl-(C₁-C₈), O-Phenyl, Phenyl, Aryl, Fluor, Chlor, OH, NO₂, SiAlkyl₃-(C₁-C₈), CN, COOH, CHO, SO₃H, NH₂, NH-Alkyl-(C₁-C₈),
N-Alkyl₂-(C₁-C₈), P-Alkyl₂-(C₁-C₈), SO₂-Alkyl-(C₁-C₆), SO-Alkyl-(C₁-C₆), CF₃, NHCO-Alkyl-(C₁-C₄), COO-Alkyl-(C₁-C₈), CONH₂, CO-Alkyl-(C₁-C₈), NHCOH, NHCOO-Alkyl-(C₁-C₄), CO-Phenyl, COO-Phenyl, CHCH-CO₂-Alkyl-(C₁-C₈), P(Phenyl)₂, CHCHCO₂H, PO-Phenyl₂, POAlkyl₂-(C₁-C₄), PO₃H₂, PO(O-Alkyl-(C₁-C₆))₂,
SO₃-Alkyl-(C₁-C₄) bedeuten, wobei Aryl die oben genannte Bedeutung hat,
und wobei R^{1a} bis R R^{7a} unabhängig voneinander Wasserstoff, (C₁-C₈)-Alkyl, O-Alkyl-(C₁-C₈),
OCO-Alkyl-(C₁-C₈), O-Phenyl, Phenyl, Aryl, Fluor, Chlor, OH, NO₂, SiAlkyl₃-(C₁-C₈), CN, COOH, CHO, SO₃H, NH₂, NH-Alkyl-(C₁-C₈), N-Alkyl₂-(C₁-C₈), P-Alkyl₂-(C₁-C₈),
SO₂-Alkyl-(C₁-C₆), SO-Alkyl-(C₁-C₆), CF₃, NHCO-Alkyl-(C₁-C₄), COO-Alkyl-(C₁-C₈), CONH₂, CO-Alkyl-(C₁-C₈), NHCOH, NHCOO-Alkyl-(C₁-C₄), CO-Phenyl, COO-Phenyl,
CHCH-CO₂-Alkyl-(C₁-C₈), P(Phenyl)₂, CHCHC02H, PO-Phenyl₂, POAlkyl₂-(C₁-C₄), PO₃H₂, PO(O-Alkyl-(C₁-C₆))₂, SO₃-Alkyl-(C₁-C₄) bedeuten, wobei Aryl die oben genannte Bedeutung hat,
und wobei Ar für einen Heteroaromaten steht,
wobei der Heteroaromat Ar einen substituierten fünf-, sechs- oder siebengliedrigen Ring mit gegebenenfalls Stickstoff-, Sauerstoff- oder Schwefelatomen im Ring bedeuten kann,
wobei an den Ring weitere aromatische, heteroaromatische oder/und aliphatische Ringe ankondensiert sein können, und
wobei der Heteroaromat Substituenten aufweisen kann,
die unabhängig voneinander Wasserstoff, (C₁-C₈)-Alkyl, OAryl, Aryl, Fluor, Chlor, OH, NO₂, CN, CO₂H, CHO, SO₃H, NH₂, NH-Alkyl-(C₁-C₁₂), N-Alkyl₂-(C₁-C₁₂), CHCHCO₂H,
NHCO-Alkyl-(C₁-C₁₂), CO-Alkyl-(C₁-C₁₂), NHCHO, COAryl, CO₂Aryl, CF₃, CONH₂, POAryl₂, POAlkyl₂-(C₁-C₁₂), SiAlkyl₃(C₁-C₁₂), O-Alkyl-(C₁-C₈), OCO-Alkyl-(C₁-C₈), O-Phenyl, Phenyl, NH-Alkyl-(C₁-C₄), COO-Alkyl-(C₁-C₈), NHCOO-Alkyl-(C₁-C₄), CO-Phenyl, COO-Phenyl, CHCH-CO₂-Alkyl-(C₁-C₈), PO-Phenyl₂, PO₃H₂, SO₃-Alkyl-(C₁-C₄), SO₂-Alkyl-(C₁-C₄),
SO-Alkyl-(C₁-C₄) sein können,
wobei Aryl die oben genannte Bedeutung hat,
und wobei
R^{8a} bis R^{10a} unabhängig voneinander Wasserstoff, CN, CO₂H, CO₂-Alkyl-(C₁-C₈), CONH₂, CONH-Alkyl-(C₁-C₄), CON-(Alkyl)₂-(C₁-C₄), Fluor, CO₂-Phenyl, Alkyl-(C₁-C₈), Phenyl, Aryl, PO(Phenyl)₂, PO[Alkyl-(C₁-C₄)]₂, CO-Phenyl, CO-Alkyl-(C₁-C₄), O-Alkyl-(C₁-C₄),
CONH-Alkyl-(C₁-C₈), CON[Alkyl-(C₁-C₈)]₂, NH-Alkyl-(C₁-C₄), PO₃H₂, SO₃H, SO₃-Alkyl-(C₁-C₄), SO₂-Alkyl-(C₁-C₄), O-Phenyl bedeuten, wobei Aryl die oben genannte Bedeutung hat,
durch Umsetzung von Aromaten, Olefinen oder/und Heteroaromaten der allgemeinen Formeln (IIa), (IIb), (llc) oder/und (IId)

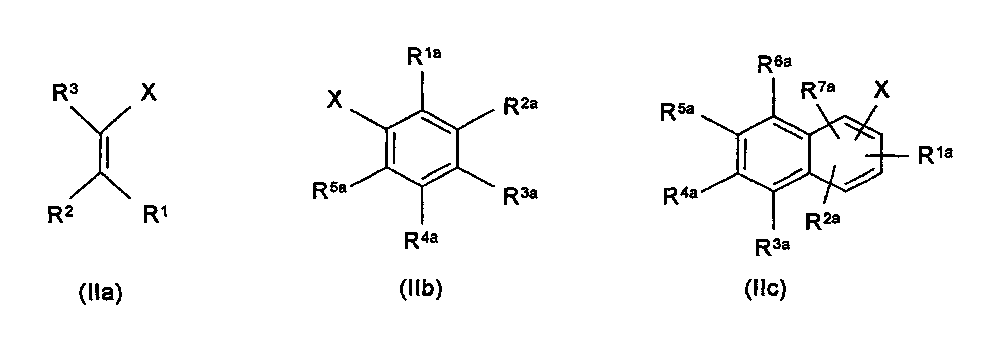

Ar-X (IId)

mit Olefinen der allgemeinen Formel (III), 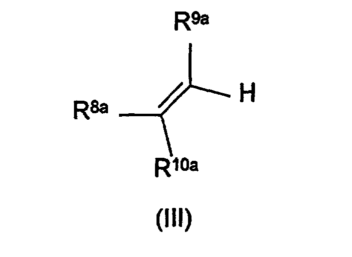 worin bei den Formeln des Typs II und III R¹ bis R³ und R^{1a} bis R^{10a} die zuvor zu den Formeln des Typs (I) angegebene Bedeutung besitzen und
wobei X Iod, Brom, Chlor, OSO₂CF₃, OSO₂Phenyl, OSO₂Tolyl, OSO₂Alkyl-(C₁-C₈) bedeutet,
das dadurch gekennzeichnet ist, daß man als Katalysator eine Mischung eines Palladium(0)-Komplexes oder eines Palladium(II)-Salzes mit Phosphitliganden der allgemeinen Formeln (IVa) oder/und (IVb) einsetzt, 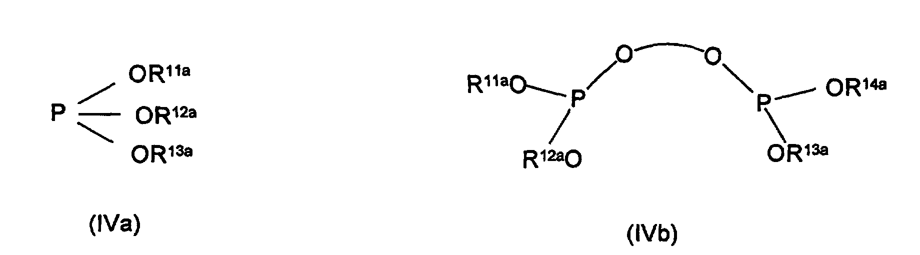 wobei die Formel (IVb) einen chelatisierenden Phosphitliganden darstellt und worin die angegebenen Reste R^{11a} bis R^{14a} gleich oder verschieden unabhängig voneinander (C₁-C₁₈)-Alkyl oder/und eine substituierte (C₁-C₁₈)-Alkyl-Gruppe mit den Substituenten Wasserstoff, O-Alkyl-(C₁-C₈), O-CO-Alkyl-(C₁-C₈), OPhenyl, Phenyl, Fluor, Chlor, OH, NO₂, CN, COOH, CHO, SO₃H, SO₂-Alkyl-(C₁-C₈), SO-Alkyl-(C₁-C₈), NH₂, NH-Alkyl-(C₁-C₈), N-Alkyl₂-(C₁-C₈), NHCO-Alkyl-(C₁-C₄), CF₃, COO-Alkyl-(C₁-C₈), CONH₂, CO-Alkyl-(C₁-C₈), NHCOH, NHCOO-Alkyl-(C₁-C₄), CO-Phenyl, COO-Phenyl, CHCH-CO₂-Alkyl-(C₁-C₈), CHCHCO₂H, PO-Phenyl₂, POAlkyl₂-(C₁-C₈)
oder die angegebenen Reste Reste R^{11a} bis R^{14a} einen Aromaten Ar' bedeuten, worin Ar' für einen substituierten Phenyl-, Naphthyl-, Anthryl-, Phenanthryl-, Biphenyl-Rest oder/und einen fünf-, sechs- oder siebengliedrigen Heteroaromaten mit gegebenenfalls Stickstoff-, Sauerstoff- oder Schwefelatomen im Ring steht,
wobei diese Aromaten bis zu 10 Substituenten enthalten können, die (C₁-C₈)-Alkyl, OAr', Ar', Fluor, Chlor, OH, NO₂, CN, CO₂H, CHO, SO₃H, NH₂, NH-Alkyl-(C₁-C₁₂), N-Alkyl₂-(C₁-C₁₂), NHCO-Alkyl-(C₁-C₁₂), CO-Alkyl-(C₁-C₁₂), NHCHO, COAr', CO₂Ar', CF₃, CONH₂, CHCHCO₂H, POAr'₂, POAlkyl₂-(C₁-C₁₂), SiAlkyl₃-(C₁-C₁₂), O-Alkyl-(C₁-C₈), OCO-Alkyl-(C₁-C₈), O-Phenyl, Phenyl, COO-Alkyl-(C₁-C₈), NHCOO-Alkyl-(C₁-C4), CO-Phenyl, COO-Phenyl, CHCH-CO₂-Alkyl-(C₁-C₈), PO-Phenyl₂, PO₃H₂, SO₃-Alkyl-(C₁-C₄), SO₂-Alkyl-(C₁-C₄), SO-Alkyl-(C₁-C₄) darstellen, wobei die Kohlenstoffbrücke des Phosphitliganden C₂ bis C₄ zwischen den beiden O-Atomen bis zu vier Substituenten aufweisen kann, die gleich oder verschieden sind und insbesondere Wasserstoff, (C₁-C₄)-Alkyl, O-Alkyl-(C₁-C₄), OH, Aryl oder/und Phenyl darstellen, wobei Aryl die oben genannte Bedeutung hat.

Insbesondere werden zur Herstellung von Verbindungen der Formel (Id) als Heterohalogenaromaten ArX der Formel (IId) substituierte Heterohalogenaromaten mit bis zu vier Heteroatomen wie N, O oder/und S im Ring eingesetzt, bevorzugt substituierte Pyridine, Pyrimidine, Oxazole, Imidazole, Pyrazine, Chinoline, Indole, Furane, Benzofurane oder/und Thiophene,
wobei die Substituenten gleich oder verschieden unabhängig voneinander Wasserstoff, (C₁-C₈)-Alkyl, OAryl, Aryl, Fluor, Chlor, OH, NO₂, CN, CO₂H, CHO, SO₃H, NH₂, NHAlkyl-(C₁-C₁₂), NAlkyl₂-(C₁-C₁₂), NHCO-Alkyl-(C₁-C₁₂), CO-Alkyl-(C₁-C₁₂), NHCHO, COAryl, CO₂Aryl, CF₃, CONH₂, POAryl₂, POAlkyl₂-(C₁-C₁₂), SiAlkyl₃(C₁-C₁₂), O-Alkyl-(C₁-C₈), OCO-Alkyl-(C₁-C₈), O-Phenyl, Phenyl, COO-Alkyl-(C₁-C₈), NHCOO-Alkyl-(C₁-C₄), CO-Phenyl, COO-Phenyl, CHCH-CO₂-Alkyl-(C₁-C₈), PO-Phenyl₂, PO₃H₂, SO₃-Alkyl-(C₁-C₄), SO₂-Alkyl-(C₁-C₄)), SO-Alkyl-(C₁-C₄) sein können, wobei Aryl die oben genannte Bedeutung hat, und
R^{8a} bis R^{10a} gleich oder verschieden unabhängig voneinander Wasserstoff, CN, CO₂H, Phenyl, CO₂Alkyl-(C₁-C₈), CONH₂, F, O-Alkyl-(C₁-C₄), Alkyl-(C₁-C₈), NH-Alkyl-(C₁-C₄) bedeuten können und
wobei X lod, Brom, Chlor, OSO₂CF₃, OSO₂Phenyl, OSO₂Tolyl, OSO₂Alkyl-(C₁-C₈) bedeuten kann.

Besonders bevorzugt ist ein Verfahren, bei dem Olefine hergestellt werden, bei denen in Formel (la) R¹ bis R³ für Wasserstoff, (C₁-C₄)-Alkyl oder/und Phenyl steht.

Das erfindungsgemäße Verfahren hat sich insbesondere für die Herstellung von Verbindungen der Formeln (la), (Ib), (Ic) oder/und (Id) bewährt, worin bedeuten:
R¹ bis R³ und R^{1a} bis R^{7a} unabhängig voneinander Wasserstoff, (C₁-C₈)-Alkyl, O-Alkyl-(C₁-C₈), O-CO-Alkyl-(C₁-C₈), N-Alkyl₂-(C₁-C₈), Phenyl, Aryl, Fluor, Chlor, NO₂, CN, COOH, CHO, SO₂-Alkyl-(C₁-C₄), NH-(C₁-C₈)-Alkyl, COO-(C₁-C₈)-Alkyl, CONH₂, CO-(C₁-C₈)-Alkyl, CO-Phenyl, oder/und PO-Phenyl₂, wobei Aryl die oben genannte Bedeutung hat,
und R^{8a} bis R^{10a} unabhängig voneinander Wasserstoff, CN, CO₂H, CO₂-Alkyl-(C₁-C₈), CONH₂, F, O-Alkyl-(C₁-C₄), Alkyl-(C₁-C₈), NH-Alkyl-(C₁-C₄), CONH-Alkyl-(C₁-C₈), CON[Alkyl-(C₁-C₈)]₂, Phenyl oder/und Aryl, wobei Aryl die oben genannte Bedeutung hat.

Besondere Bedeutung hat das Verfahren u.a. zur Herstellung von Verbindungen der Formeln (la), (Ib), (Ic) oder/und (Id), worin bedeuten:
R¹ bis R³ und R^{1a} bis R^{7a} unabhängig voneinander Wasserstoff, (C₁-C₈)-Alkyl, O-Alkyl-(C₁-C₈), Phenyl, Fluor, Chlor, NO₂, CN, COOH, CHO, COO-(C₁-C₈)-Alkyl, CONH₂, CO-(C₁-C₈)-Alkyl, CO-Phenyl oder/und PO-Phenyl₂
und R^{8a} bis R^{10a} unabhängig voneinander Wasserstoff, CN, CO₂H, CO₂-Alkyl-(C₁-C₈), Phenyl, CONH₂, F, O-Alkyl-(C₁-C₄), Alkyl-(C₁-C₈) oder/und NH-Alkyl-(C₁-C₄).

Als Lösungsmittel finden im allgemeinen inerte organische Lösungsmittel oder/und Wasser Verwendung. Besonders geeignet sind dipolar aprotische Lösungsmittel wie Dialkylsulfoxide, N,N-Dialkylamide von aliphatischen Carbonsäuren oder alkylierte Lactame. Hierbei sind Dimethylsulfoxid, N,N-Dimethylacetamid, N,N-Dimethylformamid und N-Methylpyrrolidon bevorzugt. Daneben sind als Lösungsmittel aliphatische Ether, aromatische oder aliphatische Kohlenwasserstoffe, Alkohole und Ester sowie deren Gemische geeignet.

Die Reaktion läuft vorzugsweise bei Temperaturen von 20 bis 200°C ab; in vielen Fällen hat es sich bewährt, bei Temperaturen von 60 bis 180°C, bevorzugt 100 bis 160°C, zu arbeiten. Der Druck kann mehr als 100 bar betragen, wobei üblicherweise nur bis zu einem Druck von 100 bar gearbeitet wird und vorzugsweise im Bereich des Normaldrucks bis zu 60 bar.

Da bei der Reaktion HX abgespalten wird, ist es vorteilhaft, die entstehende Säure durch Zusatz einer Base abzufangen. Dazu geeignet sind primäre, sekundäre oder/und tertiäre Amine wie Alkylamine, Dialkylamine, Trialkylamine, die alicyclisch oder/und offenkettig sein können, oder/und Alkali- oder/und Erdalkalisalze aliphatischer oder/und aromatischer Carbonsäuren, wie Acetate, Propionate, Benzoate bzw. entsprechende Carbonate, Hydrogencarbonate, Phosphate, Hydrogenphosphate oder/und Hydroxide bevorzugt von Lithium, Natrium, Kalium, Calcium, Magnesium, Cäsium.

Die eingesetzten Palladiumkatalysatoren vom Typ (IV) werden in der Regel in situ aus Palladium(II)salzen und den entsprechenden Phosphitliganden erzeugt. Sie können jedoch auch als Palladium(0)Phosphit-Komplexe direkt eingesetzt werden, ohne daß dadurch die anfängliche katalytische Aktivität gemindert wird.
Als Palladiumvorstufen können beispielsweise eingesetzt werden:
Palladium(II)acetat, Palladium(II)chlorid, Palladium(II)bromid, Lithiumtetrachloropalladat, Palladium(II)acetylacetonat, Palladium(O)-dibenzylidenaceton-Komplexe, Palladium(0)tetrakistriphenylphosphan, Palladium(0)bistri-o-tolylphosphan, Palladium(II)propionat, Palladium(II)chloridbisacetonitril, Palladium(II)-bistriphenylphosphan-dichlorid, Palladium(0)tricyclohexylphosphandiallylether-Komplex, Palladium(0)bistricyclo-hexylphosphan, Palladium(II)chloridbisbenzonitril und weitere Palladium(O)- und Palladium(II)-Komplexe. Im allgemeinen wird der Phosphitligand im vorliegenden Verfahren in Überschuß zum Palladium eingesetzt. Das Verhältnis Palladium zu Ligand beträgt vorzugsweise zwischen 1 : 2 bis 1:1000. Besonders bevorzugt werden Verhältnisse von Palladium zu Ligand von 1: 5 bis 1: 200 verwendet.

Der Einsatz eines chelatisierenden Phosphitliganden kann von Vorteil sein. Dabei weist die Kohlenstoffbrücke des Phosphitliganden C₂ bis C₄ bis zu vier Substituenten auf, die gleich oder verschieden sind und insbesondere Wasserstoff, (C₁-C₄)-Alkyl, O-Alkyl-(C₁-C₄), OH, Aryl oder/und Phenyl darstellen, wobei Aryl die oben genannte Bedeutung hat.

Bei Einsatz von Chloraromaten, Bromaromaten oder Aryltriflaten und Arylmesylaten sowie verwandten Ausgangsprodukten ist es mitunter vorteilhaft, einen Co-Katalysator zum Palladium-Phosphit-Katalysator zuzusetzen. Dieser Co-Katalysator ist ein Salz eines Halogens, insbesondere ein Halogenid der Alkalielemente oder/und Erdalkalielemente, ein Ammoniumhalogenid oder/und ein Phosphoniumhalogenid, vorzugsweise ein Bromid oder/und Chlorid. Besonders bevorzugt sind LiBr, LiCI, NaBr, KBr, CsBr, Bu₄NCl, Bu₄NBr, BzMe₃NBr, Trioctylmethylammoniumbromid, Tetraphenylphosphoniumbromid. Der Co-Katalysator wird in einer Menge von 0.01 mol% bis 100 mol% und bevorzugt von 0.1 bis 50 mol% eingesetzt. Bei verfahrenstechnischen Vorteilen kann die Reaktion auch im Co-Katalysator als Lösungsmittel durchgeführt werden.

Bei dem erfindungsgemäßen Verfahren können Turnover-Werte der Katalysatoren in der Größenordnung von 100.000 und mehr für Bromaromaten als Ausgangsmaterialien und 10.000 und mehr für Chloraromaten realisiert werden.

Aufgrund der Katalysatoraktivitäten und -stabilitäten ist es somit bei dem erfindungsgemäßen Verfahren möglich, extrem kleine Mengen an Katalysator zu verwenden, so daß die Katalysatorkosten im Vergleich zu herkömmlichen Heck-Reaktionen für den entsprechenden Prozeß nicht kostenlimitierend sind. Bei den erfindungsgemäßen Verfahren werden ausnahmsweise Gehalte an Katalysatoren von 1 bis 2 mol%, üblicherweise ≤ 1 mol%, besonders bevorzugt ≤ 0,2 mol% verwendet. Darüberhinaus sind Phosphitliganden gegenüber den bisher verwendeten Phosphanliganden einfacher herzustellen und stabiler gegenüber Oxidationsreaktionen.

Das erfindungsgemäße Verfahren ist insofern besonders überraschend und neu, da in der Vergangenheit keine Heck-Reaktionen mit Palladium-Phosphit-Katalysatoren beschrieben wurden. Das ist darauf zurückzuführen, daß bisherige mechanistische Vorstellungen zur Heck-Reaktion davon ausgingen, daß eine elektronenreiche Palladiumspezies als Katalysator benötigt wird (A. de Meijere, F.E. Meyer, Angew. Chemie 1994, 106, 2473-2506, insbesondere S. 2475). Da Phosphite elektronenärmer sind als Phosphane, wurden diese Liganden als nicht für Heck-Reaktionen geeignet angesehen (C. A. Tolman, Chem. Rev. 1977, 77, 313-348, insbesondere S. 343/344). Das erfindungsgemäße Verfahren zeigt hier erstmals, daß diese Vorstellungen falsch sind.

Die besonderen Vorteile des neuen Katalysatorsystems bestehen in der geringen Oxidationsempfindlichkeit der Phosphitliganden im Vergleich zu üblicherweise verwendeten Phosphanliganden. Darüberhinaus sind Phosphite gegenüber Phosphanen einfacher herzustellen und zu modifizieren, was sich auch im Preis der entsprechenden Liganden (z.B. 1 l Triethylphosphit DM 48 bzw 1 kg Triphenylphosphan DM 116 beim gleichen Lieferanten) niederschlägt.

Die erfindungsgemäß hergestellten Olefine können unter anderem eingesetzt werden als UV-Absorber, als Zwischenprodukte für Pharmazeutika und Agrochemikalien, als Ligandvorstufen für Metallocenkatalysatoren, als Riechstoffe, Wirkstoffe und Bausteine für Polymere.

### Beispiele:

Die nachstehenden Beispiele dienen zur Erläuterung des erfindungsmäßigen Verfahrens, ohne es darauf zu beschränken.

### Allgemeine Arbeitsvorschrift:

In einem Druckrohr (erhältlich z.B. bei Fa. Aldrich) wurden unter einer ArgonAtmosphäre 10 mmol Arylhalogenid oder bei Beispiel 14 Vinylhalogenid, 15 mmol Olefin, 12 mmol Natriumcarbonat, 2.0 mmol Tetra-n-butylammoniumbromid, eine geeignete Menge an Phosphit und Palladium(II)acetat und 500 mg Diethylenglycoldi-n-butylether (als interner Standard für die GC-Analytik) zu 10 ml trockenem N,N-Dimethylacetamid gegeben. Das Rohr wurde verschlossen und in ein 140 oder 160°C heißes Siliconölbad gehängt. Nach 24 h ließ man es auf Raumtemperatur abkühlen. Die Feststoffe wurden in 10 ml CH2CI2 und 10 ml 2 *n* Salzsäure gelöst. Die organische Phase wurde gaschromatographisch analysiert. Die Produkte wurden durch Destillation, Kristallisation aus Methanol/Aceton-Mischungen oder säulenchromatographisch (Kieselgel, Hexan/Ethylacetat-Mischungen) isoliert.
Katalysatormischung A: Triethylphosphit, P/Pd = 100: 1
Katalysatormischung B: Tris(2,4-di-tert. butylphenyl)phosphit, P/Pd = 10 : 1
Katalysatormischung C: Tri-i-propylphosphit, P/Pd = 10 : 1
Katalysatormischung D: Triphenylphosphit, P/Pd = 10 : 1
(P = Phosphitligand)

**Tabelle 1:**

| Tabelle 1: Übersicht über die erfindungsgemäßen Beispiele | | | | | | | | |
|---|---|---|---|---|---|---|---|---|
| Nr. | Halogenaromat | Olefin | Bu4NBr [mol%] | Katalysator | Pd [mol%] | Temperatur [°C] | GC-Ausbeute [%] | TON |
| 1 | 4-Chlorbenzotrifluorid | Styrol | 20 | A | 0.1 | 160 | 71 | 710 |
| 2 | 4-Chlorbenzotrifluorid | Styrol | 0.2 | A | 0.001 | 160 | 15 | 15.000 |
| 3 | 4-Chlorbenzotrifluorid | Styrol | 20 | B | 0.1 | 160 | 84 | 840 |
| 4 | 4-Chlorbenzotrifluorid | Styrol^{a)} | 20 | C | 0.1 | 160 | 51 | 510 |
| 5 | 4-Chlorbenzotrifluorid | Styrol | - | D | 0.1 | 160 | 47 | 470 |
| 6 | 4-Chlorbenzotrifluorid | N,N-Dimethylacrylsäureamid | 20 | B | 0.1 | 160 | 89 | 890 |
| 7 | 3-Chlorbenzotrifluorid | Styrol | 20 | A | 0.1 | 160 | 79 | 790 |
| 8 | 2-Chlor-5-nitrotoluol | Acrylsäure-*n*-butylester | 20 | A | 0.1 | 140 | 69 | 690 |
| 9 | 4-Bromanisol | Styrol | 20 | A | 0.1 | 140 | 66 | 660 |
| 10 | 4-Bromanisol | Styrol | - | B | 0.1 | 140 | 100 | 1.000 |
| 11 | 4-Bromanisol | Styrol | - | B | 0.001 | 140 | 30 | 30.000 |
| 12 | 2-Brom-6-methoxynaphthalin | But-3-en-2-ol^{a)} | - | B | 0.1 | 160 | 66 | 660 |
| 13 | 4-Bromacetophenon | Styrol | - | B | 0.0001 | 140 | 75 | 750.000 |
| 14 | Styrolbromid | Styrol | - | B | 0.1 | 140 | 71 | 710 |

| | | | | | | | | |
|---|---|---|---|---|---|---|---|---|
| a) Natriumacetat statt -carbonat | | | | | | | | |

Die Beispiele belegen, daß es bei dem erfindungsgemäßen Verfahren möglich ist, Ausbeuten von mindestens 47 % und in vielen Fällen von mehr als 70 % bei erhöhten turnover-Werten zu erreichen oder sehr hohe turnover-Werte bei gewissen Ausbeuten.

## Patentansprüche

1. Verfahren zur Herstellung von mono-, bi- oder/und polyfunktionellen Olefinen der Formeln (la), (Ib), (Ic) oder/und (Id), 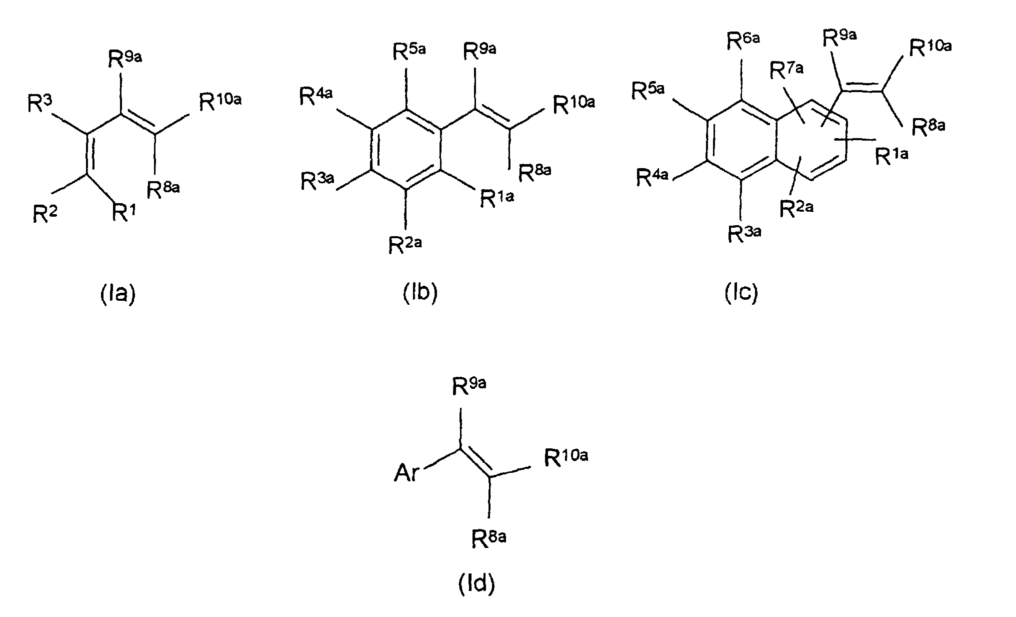 worin
R¹ bis R³ unabhängig voneinander Wasserstoff, (C₁-C₈)-Alkyl, CN, COOH, COO-Alkyl-(C₁-C₈), CO-Alkyl-(C₁-C₈), Aryl-(C₆-C₁₀), COO-Aryl-(C₆-C₁₀), CO-Aryl-(C₆-C₁₀), O-Alkyl-(C₁-C₈), O-CO-Alkyl-(C₁-C₈), N-Alkyl₂-(C₁-C₈) bedeutet und Aryl für einen bis zu 10 C-Atome enthaltenden aromatischen Rest steht, wobei dieser Rest bis zu fünf Substituenten tragen kann, die unabhängig voneinander Wasserstoff, (C₁-C₈)-Alkyl, O-Alkyl-(C₁-C₈), O-CO-Alkyl-(C₁-C₈), O-Phenyl, Phenyl, Aryl, Fluor, Chlor, OH, NO₂, SiAlkyl₃-(C₁-C₈), CN, COOH, CHO, SO₃H, NH₂, NH-Alkyl-(C₁-C₈), N-Alkyl₂-(C₁-C₈), P-Alkyl₂-(C₁-C₈), SO₂-Alkyl-(C₁-C₆), SO-Alkyl-(C₁-C₆), CF₃, NHCO-Alkyl-(C₁-C₄), COO-Alkyl-(C₁-C₈), CONH₂, CO-Alkyl-(C₁-C₈), NHCOH, NHCOO-Alkyl-(C₁-C₄), CO-Phenyl, COO-Phenyl, CHCH-CO₂-Alkyl-(C₁-C₈), P(Phenyl)₂, CHCHCO₂H, PO-Phenyl₂, POAlkyl₂-(C₁-C₄), PO₃H₂, PO(O-Alkyl-(C₁-C₆))₂, SO₃-Alkyl-(C₁-C₄) bedeuten, wobei Aryl die oben genannte Bedeutung hat, und wobei
R^{1a} bis R^{7a} unabhängig voneinander Wasserstoff, (C₁-C₈)-Alkyl, O-Alkyl-(C₁-C₈), O-CO-Alkyl-(C₁-C₈), O-Phenyl, Phenyl, Aryl, Fluor, Chlor, OH, NO₂, SiAlkyl₃-(C₁-C₈), CN, COOH, CHO, SO₃H, NH₂, NH-Alkyl-(C₁-C₈), N-N-Alkyl₂-(C₁-C₈), P-Alkyl₂-(C₁-C₈), SO₂-Alkyl-(C₁-C₆), SO-Alkyl-(C₁-C₆), CF₃, NHCO-Alkyl-(C₁-C₄), COO-Alkyl-(C₁-C₈), CONH₂, CO-Alkyl-(C₁-C₈), NHCOH, NHCOO-Alkyl-(C₁-C₄), CO-Phenyl, COO-Phenyl, CHCH-CO₂-Alkyl-(C₁-C₈), P(Phenyl)₂, CHCHCO₂H, PO-Phenyl₂, POAlkyl₂-(C₁-C₄), PO₃H₂, PO(O-Alkyl-(C₁-C₆))₂, SO₃-Alkyl-(C₁-C₄) bedeuten, wobei Aryl die oben genannte Bedeutung hat, und
Ar für einen Heteroaromaten steht, wobei der Heteroaromat Ar einen substituierten fünf-, sechs- oder siebengliedrigen Ring mit gegebenenfalls Stickstoff-, Sauerstoff- oder Schwefelatomen im Ring bedeutet, wobei an den Ring weitere aromatische, heteroaromatische oder/und aliphatische Ringe ankondensiert sein können, wobei der Heteroaromat Substituenten aufweisen kann, die unabhängig voneinander Wasserstoff, (C₁-C₈)-Alkyl, OAryl, Aryl, Fluor, Chlor, OH, NO₂, CN, CO₂H, CHO, SO₃H, NH₂, NH-Alkyl-(C₁-C₁₂), N-Alkyl₂-(C₁-C₁₂), CHCHCO₂H, NHCO-Alkyl-(C₁-C₁₂), CO-Alkyl-(C₁-C₁₂), NHCHO, COAryl, CO₂Aryl, CF₃, CONH₂, POAryl₂, POAlkyl₂-(C₁-C₁₂), SiAlkyl₃(C₁-C₁₂), O-Alkyl-(C₁-C₈), OCO-Alkyl-(C₁-C₈), O-Phenyl, Phenyl, NH-Alkyl-(C₁-C₄), COO-Alkyl-(C₁-C₈), NHCOO-Alkyl-(C₁-C₄), CO-Phenyl, COO-Phenyl, CHCH-CO₂-Alkyl-(C₁-C₈), PO-Phenyl₂, PO₃H₂, SO₃-Alkyl-(C₁-C₄), SO₂-Alkyl-(C₁-C₄), SO-Alkyl-(C₁-C₄) sein können, wobei Aryl die oben genannte Bedeutung hat, und wobei
R^{8a} bis R^{10a} unabhängig voneinander Wasserstoff, CN, CO₂H, CO₂-Alkyl-(C₁-C₈), CONH₂, CONH-Alkyl-(C₁-C₄), CON-(Alkyl)₂-(C₁-C₄), Fluor, CO₂-Phenyl, Alkyl-(C₁-C₈), Phenyl, Aryl, PO(Phenyl)₂, PO[Alkyl-(C₁-C₄)]₂, CO-Phenyl, CO-Alkyl-(C₁-C₄), O-Alkyl-(C₁-C₄), CONH-Alkyl-(C₁-C₈), CON[Alkyl-(C₁-C₈)]₂, NH-Alkyl-(C₁-C₄), PO₃H₂, SO₃H, SO₃-Alkyl-(C₁-C₄), SO₂-Alkyl-(C₁-C₄), O-Phenyl bedeuten, wobei Aryl die oben genannte Bedeutung hat,
durch Umsetzung von Aromaten, Olefinen oder/und Heteroaromaten der allgemeinen Formeln (IIa), (IIb), (IIc) oder/und (IId) 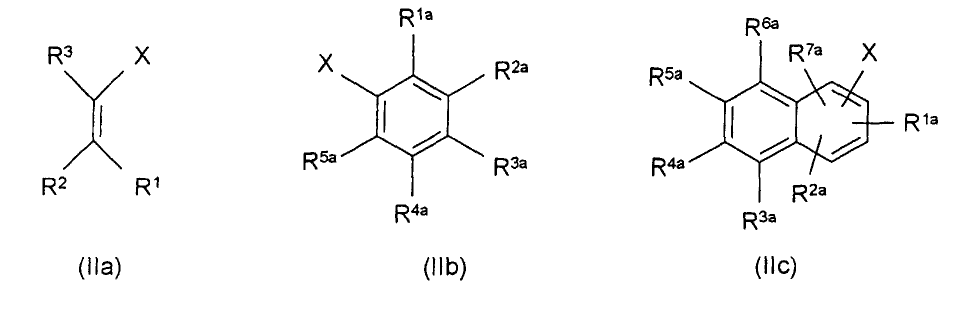
Ar-X (IId)
mit Olefinen der allgemeinen Formel (III), 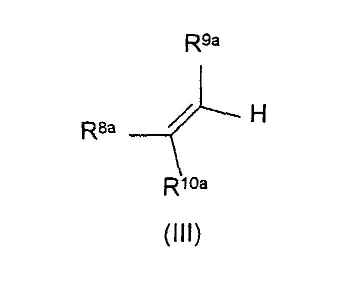 worin bei den Formeln des Typs (II) und (III) R¹ bis R³ und R^{1a} bis R^{10a} die zuvor zu den Formeln des Typs (I) angegebene Bedeutung besitzen und
wobei X Iod, Brom, Chlor, OSO₂CF₃, OSO₂Phenyl, OSO₂Tolyl, OSO₂Alkyl(C₁-C₈) bedeutet, das **dadurch gekennzeichnet ist, daß** man als Katalysator eine Mischung eines Palladium(0)-Komplexes oder eines Palladium(II)-Salzes mit Phosphitliganden der allgemeinen Formeln (IVa) oder/und (IVb) einsetzt, 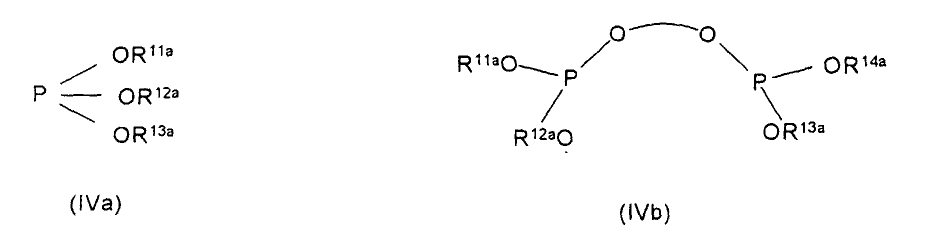 worin die angegebenen Reste R^{11a} bis R^{14a} gleich oder verschieden unabhängig voneinander (C₁-C₁₈)-Alkyl oder/und eine substituierte (C₁-C₁₈)-Alkyl-Gruppe mit den Substituenten Wasserstoff, O-Alkyl-(C₁-C₈), O-CO-Alkyl-(C₁-C₈), OPhenyl, Phenyl, Fluor, Chlor, OH, NO₂, CN, COOH, CHO, SO₃H, SO₂-Alkyl-(C₁-C₈), SO-Alkyl-(C₁-C₈), NH₂, NH-Alkyl-(C₁-C₈), N-Alkyl₂-(C₁-C₈), NHCO-Alkyl-(C₁-C₄), CF₃, COO-Alkyl-(C₁-C₈), CONH₂, CO-Alkyl-(C₁-C₈), NHCOH, NHCOO-Alkyl-(C₁-C4), CO-Phenyl, COO-Phenyl, CHCH-CO₂-Alkyl-(C₁-C₈), CHCHCO₂H, PO-Phenyl₂, POAlkyl₂-(C₁-C₈)
oder die angegebenen Reste R^{11a} bis R^{14a} einen Aromaten Ar' bedeuten, worin Ar' für einen substituierten Phenyl-, Naphthyl-, Anthryl-, Phenanthryl-, Biphenyl-Rest oder/und einen fünf-, sechs- oder siebengliedrigen Heteroaromaten mit gegebenenfalls Stickstoff-, Sauerstoff- oder Schwefelatomen im Ring steht, wobei diese Aromaten bis zu 10 Substituenten enthalten können, die (C₁-C₈)-Alkyl, OAr', Ar', Fluor, Chlor, OH, NO₂, CN, CO₂H, CHO, SO₃H, NH₂, NH-Alkyl-(C₁-C₁₂), N-Alkyl₂-(C₁-C₁₂), NHCO-Alkyl-(C₁-C₁₂), CO-Alkyl-(C₁-C₁₂), NHCHO, COAr', CO₂Ar', CF₃, CONH₂, CHCHCO₂H, POAr'₂, POAlkyl₂-(C₁-C₁₂), SiAlkyl₃-(C₁-C₁₂), O-Alkyl-(C₁-C₈), OCO-Alkyl-(C₁-C₈), O-Phenyl, Phenyl, COO-Alkyl-(C₁-C₈), NHCOO-Alkyl-(C₁-C₄), CO-Phenyl, COO-Phenyl, CHCH-CO₂-Alkyl-(C₁-C₈), PO-Phenyl₂, PO₃H₂, SO₃-Alkyl-(C₁-C₄), SO₂-Alkyl-(C₁-C₄), SO-Alkyl-(C₁-C₄) darstellen, wobei die Formel (IVb) einen chelatisierenden Phosphitliganden mit einer C₂ bis C₄ Kohlenstoffbrücke zwichen den beiden O-Atomen darstellt, wobei die Kohlenstoffbrücke des Phosphitliganden bis zu vier Substituenten aufweisen kann, die gleich oder verschieden sind und insbesondere Wasserstoff, (C₁-C₄)-Alkyl, O-Alkyl-(C₁-C₄), OH, Aryl oder/und Phenyl darstellen, wobei Aryl die oben genannte Bedeutung hat.

2. Verfahren nach Anspruch 1 zur Herstellung von Verbindungen der Formel (Id), **dadurch gekennzeichnet, daß** als Heterohalogenaromaten ArX der Formel (IId) substituierte Heterohalogenaromaten mit bis zu vier Heteroatomen wie N, O oder/und S im Ring eingesetzt werden, bevorzugt substituierte Pyridine, Pyrimidine, Oxazole, Imidazole, Pyrazine, Chinoline, Indole, Furane, Benzofurane oder/und Thiophene, wobei die Substituenten gleich oder verschieden unabhängig voneinander Wasserstoff, (C₁-C₈)-Alkyl, OAryl, Aryl, Fluor, Chlor, OH, NO₂, CN, CO₂H, CHO, SO₃H, NH₂, NHAlkyl-(C₁-C₁₂), NAlkyl₂-(C₁-C₁₂), NHCO-Alkyl-(C₁-C₁₂), COAlkyl-(C₁-C₁₂), NHCHO, COAryl, CO₂Aryl, CF₃, CONH₂, POAryl₂, POAlkyl₂-(C₁-C₁₂), SiAlkyl₃(C₁-C₁₂), O-Alkyl-(C₁-C₈), OCO-Alkyl-(C₁-C₈), O-Phenyl, Phenyl, COO-Alkyl-(C₁-C₈), NHCOO-Alkyl-(C₁-C₄), CO-Phenyl, COO-Phenyl, CHCH-CO₂-Alkyl-(C₁-C₈), PO-Phenyl₂, PO₃H₂, SO₃-Alkyl-(C₁-C₄), SO₂-Alkyl-(C₁-C₄)), SO-Alkyl-(C₁-C₄) sein können, wobei Aryl die oben genannte Bedeutung hat, und R^{8a} bis R^{10a} gleich oder verschieden unabhängig voneinander Wasserstoff, CN, CO₂H, Phenyl, CO₂Alkyl-(C₁-C₈), CONH₂, F, O-Alkyl-(C₁-C₄), Alkyl-(C₁-C₈), NH-Alkyl-(C₁-C₄) bedeuten.

3. Verfahren nach Anspruch 1 oder 2, **dadurch gekennzeichnet, daß** Olefine hergestellt werden, bei denen in Formel (la) R¹ bis R³ für Wasserstoff (C₁-C₄)-Alkyl oder/und Phenyl steht.

4. Verfahren zur Herstellung von Verbindungen der Formeln (Ia), (Ib), (Ic) oder/und (Id) nach einem der Ansprüche 1 bis 3, **dadurch gekennzeichnet, daß** R¹ bis R³ und R^{1a} bis R^{7a} unabhängig voneinander Wasserstoff, (C₁-C₈)-Alkyl, O-Alkyl-(C₁-C₈), O-CO-Alkyl-(C₁-C₈), Phenyl, Aryl, Fluor, Chlor, NO₂, CN, COOH, CHO, SO₂-Alkyl-(C₁-C₄), NH-(C₁-C₈)-Alkyl, N[(C₁-C₈)Alkyl)]₂, COO-(C₁-C₈)-Alkyl, CONH₂, CO-(C₁-C₈)-Alkyl, CO-Phenyl oder/und PO(Phenyl)₂, wobei Aryl die oben genannte Bedeutung hat, und daß
R^{8a} bis R^{10a} unabhängig voneinander Wasserstoff, CN, CO₂H, CO₂-Alkyl-(C₁-C₈), CONH₂, F, O-Alkyl-(C₁-C₄), Alkyl-(C₁-C₈), NH-Alkyl-(C₁-C₄), CONH-Alkyl-(C₁-C₈), CON[Alkyl-(C₁-C₈)]₂, Phenyl oder/und Aryl bedeuten, wobei Aryl die oben genannte Bedeutung hat.

5. Verfahren nach einem der Ansprüche 1 bis 4, **dadurch gekennzeichnet, daß** als Lösungsmittel inerte organische Lösungsmittel oder/und Wasser verwendet werden, vorzugsweise dipolar aprotische Lösungsmittel, aliphatische Ether, aromatische oder aliphatische Kohlenwasserstoffe, Alkohole und Wasser sowie deren Gemische, insbesondere Dialkylsulfoxide, N,N-Dialkylamide von aliphatischen Carbonsäuren oder alkylierte Lactame, speziell Dimethylsulfoxid, N,N-Dimethylacetamid, N,N-Dimethylformamid, N-Methylpyrrolidon.

6. Verfahren nach einem der vorstehenden Ansprüche, **dadurch gekennzeichnet, daß** die Reaktion bei Temperaturen von 20 bis 200°C abläuft; vorzugsweise bei 60 bis 180°C, besonders bevorzugt 100 bis 160°C, wobei der Druck bis zu 100 bar betragen kann.

7. Verfahren nach einem der vorstehenden Ansprüche, **dadurch gekennzeichnet, daß** HX, das bei der Reaktion abgespalten wird, durch Zusatz einer Base abgefangen wird, vorzugsweise mit primären, sekundären oder/und tertiären Aminen, insbesondere mit Alkylaminen, Dialkylaminen, Trialkylaminen, die bevorzugt alicyclisch oder/und offenkettig sind, oder/und mit Alkali- oder/und Erdalkalisalzen aliphatischer oder/und aromatischer Carbonsäuren, insbesondere mit Acetaten, Propionaten, Benzoaten bzw. entsprechenden Carbonaten, Hydrogencarbonaten, Phosphaten, Hydrogenphosphaten oder/und Hydroxiden bevorzugt von Lithium, Natrium, Kalium, Calcium, Magnesium, Cäsium.

8. Verfahren nach einem der vorstehenden Ansprüche, **dadurch gekennzeichnet, daß** die eingesetzten Palladiumkatalysatoren (IV) in situ aus Palladium(II)salzen und den entsprechenden Phosphitliganden erzeugt werden.

9. Verfahren nach einem der vorstehenden Ansprüche, **dadurch gekennzeichnet, daß** die eingesetzten Palladiumkatalysatoren (IV) als Palladium(0)Phosphit-Komplexe direkt eingesetzt werden.

10. Verfahren nach einem der vorstehenden Ansprüche, **dadurch gekennzeichnet, daß** als Palladiumvorstufen Palladium(II)acetat, Palladium(II)chlorid, Palladium(II)bromid, Lithiumtetrachloropalladat, Palladium(II)acetylacetonat, Palladium(0)dibenzylidenaceton-Komplexe, Palladium(0)-tetrakistriphenylphosphan, Palladium(0)bistri-o-tolylphosphan, Palladium(II)propionat, Palladium(II)chlorid-bisacetonitril, Palladium(II)bistriphenylphosphandichlorid, Palladium(0)tricyclohexylphosphandiallylether-Komplex, Palladium(0)bistricyclohexyl-phosphan, Palladium(II)chloridbisbenzonitril oder/und weitere Palladium(O)- und Palladium(II)-Komplexe eingesetzt werden.

11. Verfahren nach einem der vorstehenden Ansprüche, **dadurch gekennzeichnet, daß** ein chelatisierender Phosphitligand der Formel (IVb) eingesetzt wird, dessen Kohlenstoffbrücke C₂ bis C₄ bis zu vier Substituenten gleich oder verschieden aufweist, insbesondere Wasserstoff, (C₁-C₄)-Alkyl, OAlkyl-(C₁-C₄), OH, Aryl oder/und Phenyl, wobei Aryl die oben genannte Bedeutung hat.

12. Verfahren nach einem der vorstehenden Ansprüche, **dadurch gekennzeichnet, daß** der Katalysator in einem Verhältnis Palladium : Ligand zwischen 1 : 2 und 1 : 1000 eingesetzt wird, vorzugsweise zwischen 1: 5 und 1 : 200.

13. Verfahren nach einem der vorstehenden Ansprüche, **dadurch gekennzeichnet, daß** ein Co-Katalysator zum Palladium-Phosphit-Katalysator zugesetzt wird, vorzugsweise ein Salz eines Halogens, insbesondere ein Halogenid der Alkalielemente oder/und Erdalkali-elemente, ein Ammoniumhalogenid oder/und ein Phosphoniumhalogenid, vorzugsweise ein Bromid oder/und Chlorid, vor allem LiBr, LiCl, NaBr, KBr, CsBr, Bu₄NCl, Bu₄NBr, BzMe₃NBr, Tetraphenylphosphoniumbromid, Trioctylmethylammoniumbromid.

14. Verfahren nach Anspruch 13, **dadurch gekennzeichnet, daß** der Co-Katalysator in einer Menge von 0.01 mol% bis 100 mol% zugegeben wird, vorzugsweise von 0.1 bis 50 mol%.

15. Verfahren nach einem der vorstehenden Ansprüche, **dadurch gekennzeichnet, daß** Gehalte an Katalysatoren von ≤ 2 mol%, vorzugsweise von ≤ 1 mol%, besonders bevorzugt von ≤ 0,2 mol% verwendet werden.

## Claims

1. Process for the preparation of mono-, bi- or/and polyfunctional olefins of formulae (Ia), (Ib), (Ic) or/and (Id) 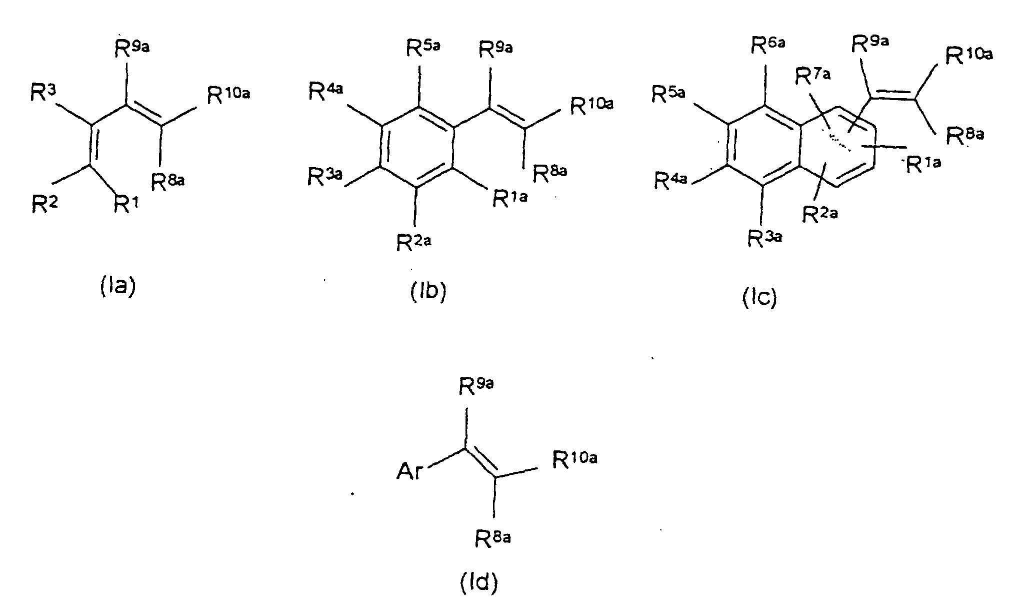 in which
R¹ to R³ each independently of the others represents hydrogen, (C₁-C₈)-alkyl, CN, COOH, COO-(C₁-C₈)-alkyl, CO-(C₁-C₈)-alkyl, (C₆-C₁₀)-aryl, COO-(C₆-C₁₀)-aryl, CO-(C₆-C₁₀)-aryl, O-(C₁-C₈)-alkyl, O-CO-(C₁-C₈)-alkyl, N-(C₁-C₈)-alkyl₂, and aryl represents an aromatic radical containing up to 10 carbon atoms, wherein that radical may carry up to five substituents which, independently of one another, represent hydrogen, (C₁-C₈)-alkyl, O-(C₁-C₈)-alkyl, O-CO-(C₁-C₈)-alkyl, O-phenyl, phenyl, aryl, fluorine, chlorine, OH, NO₂, Si-(C₁-C₈)-alkyl₃, CN, COOH, CHO, SO₃H, NH₂, NH-(C₁-C₈)-alkyl, N- (C₁-C₈)-alkyl₂, P-(C₁-C₈)-alkyl₂, SO₂-(C₁-C₆)-alkyl, SO-(C₁-C₆)-alkyl, CF₃, NHCO-(C₁-C₄)-alkyl, COO-(C₁-C₈)-alkyl, CONH₂, CO-(C₁-C₈)-alkyl, NHCOH, NHCOO-(C₁-C₄)-alkyl, CO-phenyl, COO-phenyl, CHCH-CO₂-(C₁-C₈)-alkyl, P(phenyl)₂, CHCHCO₂H, PO-phenyl₂, PO-(C₁-C₄)-alkyl₂, PO₃H₂, PO(O-(C₁-C₆)-alkyl)₂, SO₃-(C₁-C₄)-alkyl, wherein aryl is as defined above, and in which
R^{1a} to R^{7a} each independently of the others represents hydrogen, (C₁-C₈)-alkyl, O-(C₁-C₈)-alkyl, O-CO-(C₁-C₈)-alkyl, O-phenyl, phenyl, aryl, fluorine, chlorine, OH, NO₂, Si-(C₁-C₈)-alkyl₃, CN, COOH, CHO, SO₃H, NH₂, NH-(C₁-C₈)-alkyl, N-(C₁-C₈)-alkyl₂, P-(C₁-C₈)-alkyl₂, SO₂-(C₁-C₆)-alkyl, SO-(C₁-C₆)-alkyl, CF₃, NHCO-(C₁-C₄)-alkyl, COO-(C₁-C₈)-alkyl, CONH₂, CO-(C₁-C₈) -alkyl, NHCOH, NHCOO-(C₁-C₄)-alkyl, CO-phenyl, COO-phenyl, CHCH-CO₂-(C₁-C₈)-alkyl, P(phenyl)₂, CHCHCO₂H, PO-phenyl₂, PO- (C₁-C₄) -alkyl₂, PO₃H₂, PO(O-(C₁-C₆)-alkyl)₂, SO₃-(C₁-C₄)-alkyl, wherein aryl is as defined above, and
Ar represents a heteroaromatic radical, wherein the heteroaromatic radical Ar represents a substituted five-, six- or seven-membered ring optionally having nitrogen, oxygen or sulfur atoms in the ring, wherein further aromatic, heteroaromatic or/and aliphatic rings may be fused on the ring, wherein the heteroaromatic radical may contain substituents which, independently of one another, may be hydrogen, (C₁-C₈)-alkyl, O-aryl, aryl, fluorine, chlorine, OH, NO₂, CN, CO₂H, CHO, SO₃H, NH₂, NH-(C₁-C₁₂)-alkyl, N-(C₁-C₁₂)-alkyl₂, CHCHCO₂H, NHCO-(C₁-C₁₂)-alkyl, CO-(C₁-C₁₂)-alkyl, NHCHO, CO-aryl, CO₂-aryl, CF₃, CONH₂, PO-aryl₂, PO-(C₁-C₁₂)-alkyl₂, Si- (C₁-C₁₂)-alkyl₃, O-(C₁-C₈)-alkyl, OCO-(C₁-C₈)-alkyl, O-phenyl, phenyl, NH-(C₁-C₄)-alkyl, COO-(C₁-C₈)-alkyl, NHCOO-(C₁-C₄)-alkyl, CO-phenyl, COO-phenyl, CHCH-CO₂- (C₁-C₈) -alkyl, PO-phenyl₂, PO₃H₂, SO₃-(C₁-C₄)-alkyl, SO₂- (C₁-C₄)-alkyl, SO- (C₁-C₄) -alkyl, wherein aryl is as defined above, and in which
R^{8a} to R^{10a} each independently of the others represents hydrogen, CN, CO₂H, CO₂-(C₁-C₈)-alkyl, CONH₂, CONH-(C₁-C₄)-alkyl, CON-(C₁-C₄)-alkyl₂, fluorine, CO₂-phenyl, (C₁-C₈)-alkyl, phenyl, aryl, PO(phenyl)₂, PO[(C₁-C₄)-alkyl]₂, CO-phenyl, CO-(C₁-C₄)-alkyl, O-(C₁-C₄)-alkyl, CONH-(C₁-C₈)-alkyl, CON[(C₁-C₈)-alkyl]₂, NH-(C₁-C₄)-alkyl, PO₃H₂, SO₃H, SO₃-(C₁-C₄)-alkyl, SO₂-(C₁-C₄)-alkyl, O-phenyl, wherein aryl is as defined above,
by reaction of aromatic compounds, olefins or/and heteroaromatic compounds of the general formulae (IIa), (IIb), (IIc) or/and (IId) 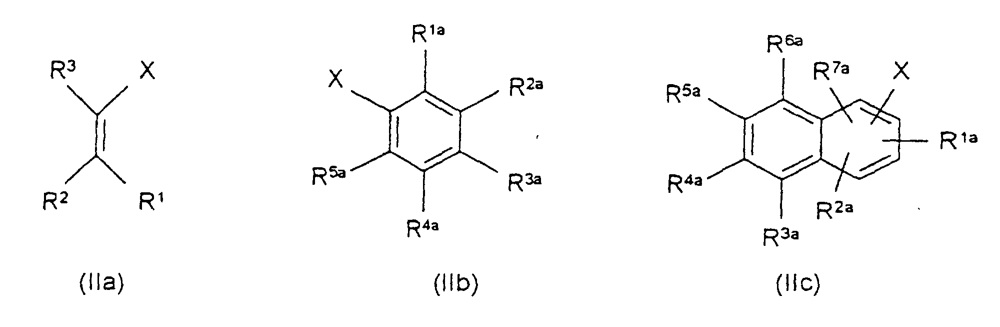
Ar - X (IId)
with olefins of the general formula (III) 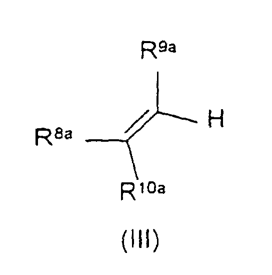 wherein in the formulae of type (II) and (III) R¹ to R³ and R^{1a} to R^{10a} are as defined above for the formulae of type (I), and
wherein X represents iodine, bromine, chlorine, OSO₂CF₃, OSO₂phenyl, OSO₂tolyl, OSO₂(C₁-C₈)-alkyl, which process is **characterised in that** there is used as catalyst a mixture of a palladium(0) complex or of a palladium(II) salt with phosphite ligands of the general formulae (IVa) or/and (IVb) 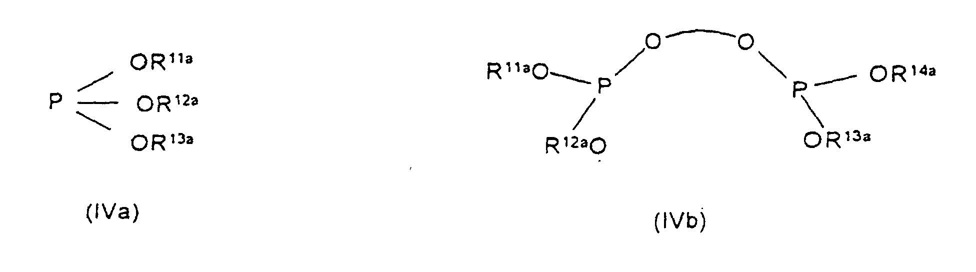 wherein the indicated radicals R^{11a} to R^{14a} are identical or different and each independently of the others represents (C₁-C₁₈)-alkyl or/and a (C₁-C₁₈)-alkyl group substituted by the substituents hydrogen, O-(C₁-C₈)-alkyl, O-CO-(C₁-C₈)-alkyl, O-phenyl, phenyl, fluorine, chlorine, OH, NO₂, CN, COOH, CHO, SO₃H, SO₂-(C₁-C₈)-alkyl, SO-(C₁-C₈)-alkyl, NH₂, NH-(C₁-C₈)-alkyl, N-(C₁-C₈)-alkyl₂, NHCO- (C₁-C₄)-alkyl, CF₃, COO-(C₁-C₈)-alkyl, CONH₂, CO-(C₁-C₈)-alkyl, NHCOH, NHCOO-(C₁-C₄)-alkyl, CO-phenyl, COO-phenyl, CHCH-CO₂-(C₁-C₈)-alkyl, CHCHCO₂H, PO-phenyl₂, PO-(C₁-C₈)-alkyl₂
or the indicated radicals R^{11a} to R^{14a} represent an aromatic radical Ar', wherein Ar' represents a substituted phenyl, naphthyl, anthryl, phenanthryl, biphenyl radical or/and a five-, six- or seven-membered heteroaromatic radical optionally having nitrogen, oxygen or sulfur atoms in the ring, wherein those aromatic radicals may contain up to 10 substituents which represent (C₁-C₈)-alkyl, OAr', Ar', fluorine, chlorine, OH, NO₂, CN, CO₂H, CHO, SO₃H, NH₂, NH-(C₁-C₁₂)-alkyl, N-(C₁-C₁₂)-alkyl₂, NHCO-(C₁-C₁₂)-alkyl, CO-(C₁-C₁₂)-alkyl, NHCHO, COAr', CO₂Ar', CF₃, CONH₂, CHCHCO₂H, POAr'₂, PO-(C₁-C₁₂)-alkyl₂, Si-(C₁-C₁₂)-alkyl₃, O-(C₁-C₈)-alkyl, OCO-(C₁-C₈)-alkyl, O-phenyl, phenyl, COO-(C₁-C₈)-alkyl, NHCOO-(C₁-C₄)-alkyl, CO-phenyl, COO-phenyl, CHCH-CO₂-(C₁-C₈)-alkyl, PO-phenyl₂, PO₃H₂, SO₃-(C₁-C₄)-alkyl, SO₂-(C₁-C₄)-alkyl, SO-(C₁-C₄)-alkyl, wherein formula (IVb) represents a chelating phosphite ligand having a C₂ to C₄ carbon bridge between the two O atoms, wherein the carbon bridge of the phosphite ligand may contain up to four substituents which are identical or different and represent especially hydrogen, (C₁-C₄) -alkyl, O-(C₁-C₄)-alkyl, OH, aryl or/and phenyl, wherein aryl is as defined above.

2. Process according to claim 1 for the preparation of compounds of formula (Id), **characterised in that** there are used as the heterohaloaromatic compounds ArX of formula (IId) substituted heterohaloaromatic compounds having up to four hetero atoms such as N, O or/and S in the ring, preferably substituted pyridines, pyrimidines, oxazoles, imidazoles, pyrazines, quinolines, indoles, furans, benzofurans or/and thiophenes, wherein the substituents are identical or different and, independently of one another, may be hydrogen, (C₁-C₈)-alkyl, O-aryl, aryl, fluorine, chlorine, OH, NO₂, CN, CO₂H, CHO, SO₃H, NH₂, NH- (C₁-C₁₂) -alkyl, N- (C₁-C₁₂) -alkyl₂, NHCO- (C₁-C₁₂)-alkyl, CO-(C₁-C₁₂)-alkyl, NHCHO, CO-aryl, CO₂-aryl, CF₃, CONH₂, PO-aryl₂, PO-(C₁-C₁₂)-alkyl₂, Si-(C₁-C₁₂)-alkyl₃, O-(C₁-C₈)-alkyl, OCO-(C₁-C₈)-alkyl, O-phenyl, phenyl, COO-(C₁-C₈)-alkyl, NHOOO-(C₁-C₄)-alkyl, CO-phenyl, COO-phenyl, CHCH-CO₂-(C₁-C₈)-alkyl, PO-phenyl₂, PO₃H₂, SO₃- (C₁-C₄)-alkyl, SO₂-(C₁-C₄-alkyl, SO-(C₁-C₄)-alkyl, wherein aryl is as defined above, and R^{8a} to R^{10a} are identical or different and each independently of the others represents hydrogen, CN, CO₂H, phenyl, CO₂-(C₁-C₈)-alkyl, CONH₂, F, O-(C₁-C₄)-alkyl, (C₁-C₈)-alkyl, NH- (C₁-C₄)-alkyl.

3. Process according to claim 1 or 2, **characterised in that** olefins in which R¹ to R³ in formula (Ia) represent hydrogen, (C₁-C₄)-alkyl or/and phenyl are prepared.

4. Process for the preparation of compounds of formulae (Ia), (Ib), (Ic) or/and (Id) according to any one of claims 1 to 3, **characterised in that** R¹ to R³ and R^{1a} to R^{7a} each independently of the others represents hydrogen, (C₁-C₈)-alkyl, O-(C₁-C₈)-alkyl, O-CO-(C₁-C₈)-alkyl, phenyl, aryl, fluorine, chlorine, NO₂, CN, COOH, CHO, SO₂-(C₁-C₄)-alkyl, NH-(C₁-C₈)-alkyl, N [(C₁-C₈)-alkyl]₂, COO-(C₁-C₈)-alkyl, CONH₂, CO-(C₁-C₈)-alkyl, CO-phenyl or/and PO(phenyl)₂, wherein aryl is as defined above, and **in that** R^{8a} to R^{10a} each independently of the others represents hydrogen, CN, CO₂H, CO₂-(C₁-C₈)-alkyl, CONH₂, F, O-(C₁-C₄)-alkyl, (C₁-C₈)-alkyl, NH- (C₁-C₄)-alkyl, CONH-(C₁-C₈)-alkyl, CON[(C₁-C₈)-alkyl]₂, phenyl or/and aryl, wherein aryl is as defined above.

5. Process according to any one of claims 1 to 4, **characterised in that** there are used as solvent inert organic solvents or/and water, preferably dipolar aprotic solvents, aliphatic ethers, aromatic or aliphatic hydrocarbons, alcohols and water, as well as mixtures thereof, especially dialkyl sulfoxides, N,N-dialkylamides of aliphatic carboxylic acids or alkylated lactams, especially dimethyl sulfoxide, N,N-dimethylacetamide, N,N-dimethylformamide, N-methylpyrrolidone.

6. Process according to any one of the preceding claims, **characterised in that** the reaction takes place at temperatures from 20 to 200°C; preferably at from 60 to 180°C, particularly preferably from 100 to 160°C, it being possible for the pressure to be up to 100 bar.

7. Process according to any one of the preceding claims, **characterised in that** HX, which is cleaved during the reaction, is captured by addition of a base, preferably with primary, secondary or/and tertiary amines, especially with alkylamines, dialkylamines, trialkylamines, which are preferably alicyclic or/and open-chained, or/and with alkali or/and alkaline earth salts of aliphatic or/and aromatic carboxylic acids, especially with acetates, propionates, benzoates or corresponding carbonates, hydrogen carbonates, phosphates, hydrogen phosphates or/and hydroxides, preferably of lithium, sodium, potassium, calcium, magnesium, caesium.

8. Process according to any one of the preceding claims, **characterised in that** the palladium catalysts (IV) that are used are produced *in situ* from palladium(II) salts and the corresponding phosphite ligands.

9. Process according to any one of the preceding claims, **characterised in that** the palladium catalysts (IV) that are used are used directly in the form of palladium(0) phosphite complexes.

10. Process according to any one of the preceding claims, **characterised in that** there are used as palladium precursors palladium(II) acetate, palladium(II) chloride, palladium(II) bromide, lithium tetrachloropalladate, palladium(II) acetylacetonate, palladium(0) dibenzylideneacetone complexes, palladium(0) tetrakistriphenylphosphane, palladium(0) bistri-o-tolylphosphane, palladium(II) propionate, palladium(II) chloride bisacetonitrile, palladium(II) bistriphenylphosphane dichloride, palladium(0) tricyclohexylphosphane diallyl ether complex, palladium(0) bistricyclohexylphosphane, palladium(II) chloride bisbenzonitrile or/and further palladium(0) and palladium(II) complexes.

11. Process according to any one of the preceding claims, **characterised in that** there is used a chelating phosphite ligand of formula (IVb) whose C₂ to C₄ carbon bridge contains up to four identical or different substituents, especially hydrogen, (C₁-C₄)-alkyl, O-(C₁-C₄)-alkyl, OH, aryl or/and phenyl, wherein aryl is as defined above.

12. Process according to any one of the preceding claims, **characterised in that** the catalyst is used in a palladium:ligand ratio of from 1:2 to 1:1000, preferably from 1:5 to 1:200.

13. Process according to any one of the preceding claims, **characterised in that** a co-catalyst is added to the palladium phosphite catalyst, preferably a salt of a halogen, especially a halide of the alkali elements or/and alkaline earth elements, an ammonium halide or/and a phosphonium halide, preferably a bromide or/and chloride, especially LiBr, LiCl/ NaBr, KBr, CsBr, Bu₄NCl, Bu₄NBr, BzMe₃NBr, tetraphenylphosphonium bromide, trioctylmethylammonium bromide.

14. Process according to claim 13, **characterised in that** the co-catalyst is added in an amount of from 0.01 mol% to 100 mol%, preferably from 0.1 to 50 mol%.

15. Process according to any one of the preceding claims, **characterised in that** catalyst contents of ≤ 2 mol%, preferably of ≤ 1 mol%, particularly preferably of ≤ 0.2 mol% are used.

## Revendications

1. Procédé de préparation d'oléfines mono, bi et/ou polyfonctionnclles des formules (Ia), (Ib, (Ic) et/ou (Id) 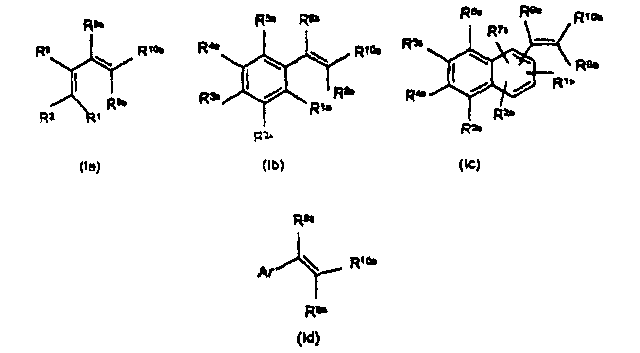 dans laquelle R¹ à R³, indépendamment les uns des autres, signifient un alkyle en C₁-C₈, CN, COOH, COO-alkyle en C₁₋C₈, CO-alkyle en C₁₋C₈, aryle en C₆₋C₁₀, COO-aryle en C₆₋C₁₀, CO-aryle en C₆₋C₁₀, O-alkyle en C₁-C₈, O-CO-alkyle en C₁₋C₈, N-alkyle)₂ en C₁₋C₈), et aryle représente un reste aromatique contenant jusqu'à 10 atomes de carbone, ce reste pouvant porter jusqu'à cinq substituants qui signifient indépendamment l'un de l'autre de l'hydrogène, un allyle en C₁₋C₈, un O-alkyle cn C₁₋C₈, un OCO-alkyle en C₁-C₈, CN COOH, CHO, SO₃H, NH₂, NH-alkyle en C₁₋C₈, N-(alkyle)₂ en C₁-C₈, P-(alkyle)₂ en C₁₋C₈, SO₂-alkyle en C₁₋C₈, SO-alkyle en C₁₋C₈, CF₃, NHCOalkyle en C₁₋C₄, COOalkyle en C₁₋C₈, CONH₂, CO-alkyle en C₁₋C₈, NHCOH, NHCOOalkyle en C₁₋C₄, CO-phényle, COO-phényle, CH CH-CO₂-alkyle C₁₋C₈, P(phényle)₂, CH CH CO₂H, PO(phényle)₂, PO(alkyle)₂ en C₁₋C₄, PO₃H₂, PO(O-alkyle en C₁-C₆)₂, SO₃alkyle en C₁₋C₄, aryle ayant la signification citée ci-dessus, et dans laquelle R^{1a} à R^{7a} indépendamment les uns des autres signifient de l'hydrogène (C₁₋C₈)-Alkyl, O-Alkyl-(C₁₋C₈), O-CO-Alkyl-(C₁₋C₈),
O-Phényl, Phényl, Aryl, Fluor, Chlore, OH, NO₂, SiAlkyl₃-(C₁₋C₈), CN, COOH, CHO, SO₃H, NH₂, NH-Alkyl-(C₁₋C₈), N-Alkyl₂-(C₁₋C₈), P-Alkyl-(C₁-C₈), SO₂-Alkyl-(C₁₋C₆), SO-Alkyl-(C₁₋C₆), CF₃, NHCO-Alkyl-(C₁₋C₄), COO-Alkyl-(C₁₋C₈), CONH₂, CO-Alkyl-(C₁-C₈), NHCOH, NHCOO-Alkyl-(C₁₋C₄), CO-Phényl, COO-Phényl, CHCH-CO₂-Alkyl(C₁-C₈), P-(Phényl)₂, CHCHCO₂H, PO-Phényl₂, POAlkyl₂-(C₁₋C₄), PO₃H₂, PO(O-Alkyl-(C₁-C₆))₂, SO₃-Alkyl-(C₁₋C₄), dans laquelle aryle a les significations citées ci-dessus et
Ar signifie un hétéro-aromatique dans lequel l'hétéro-aromatique Ar peut être un cycle à cinq, six ou sept maillons ayant éventuellement des atomes d'azote, d'oxygène ou de soufre dans le cycle, dans lequel sur le cycle d'autres cycles aromatiques, hétéro-aromatiques, et/ou aliphatiques peuvent être condensés, le composé hétéro-aromatique pouvant posséder des substituants qui, indépendamment l'un de l'autre, peuvent être de l'hydrogène, (C₁₋C₈)-Alkyl, OAryl, Aryl, Fluor, Chlore, OH, NO₂, CN, CO₂H, CHO, SO₃H, NH₂, NH-Alkyl-(C₁₋C₁₂), N-Alkyl₂-(C₁₋C₁₂), CHCHCO₂H, NHCO-Alkyl-(C₁-C₁₂), CO-Alkyl-(C₁₋C₁₂), NHCHO, COAryl, CO₂Aryl, CF₃, CONH₂, POAryl₂, POAlkyl₂-(C₁-C₁₂), SiAlkyl₃(C₁₋C₁₂), O-Alkyl-(C₁₋C₈), OCO-Alkyl-(C₁₋C₈), O-Phényl, Phényl, NH-Alkyl (C₁₋C₄), COO-Alkyl-(C₁₋C₈), NHCOO-Alkyl₂-(C₁₋C₄), CO-Phényl, COO-Phényl, CHCH-CO₂-Alkyl-(C₁₋C₈), PO-Phényl₂, PO₃H₂, SO₃-Alkyl-(C₁₋C₄), SO₂-Alkyl-(C₁₋C₄) ou SO-Alkyl-(C₁₋C₄),
aryle ayant la signification citée ci-dessus, et R^{8a} R^{10a}, indépendamment l'un de l'autre signifient de l'hydrogène CN, CO₂H, CO₂ (Alkyl en C₁-C₈), CONH₂, CONH-Alkyl-(C₁-C₄), CON-(Alkyl)₂-(C₁-C₄), Fluor, CO₂-Phényl, Alkyl-(C₁₋C₈), Phényl, Aryl, PO(Phényl)₂, PO[Alkyl-(C₁-C₄)]₂, CO-Phényl, CO-Alkyl-(C₁-C₄), O-Alkyl-(C₁-C₄), CONH-Alkyl-(C₁-C₈), CON[Alkyl-(C₁-C₈)]₂, NH-Alkyl-(C₁-C₄), PO₃H₂, SO₃H, SO₃-Alkyl-en C₁-C₄, SO₂-Alkyl-(C₁-C₄) ou O-Phényl, aryle ayant la signification citée ci-dessus, par mise en réaction de composés aromatiques d'oléfines et/ou de composés hétéro-aromatiques des formules générales (IIa), (IIb), (Ile), et/ou (IId) 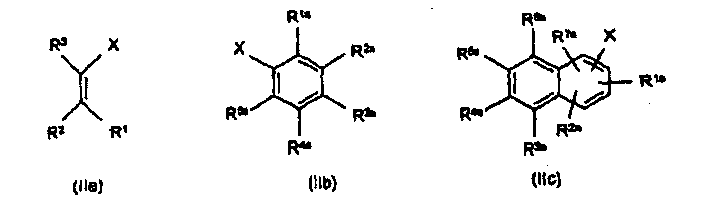
Ar - X (IId)
avec des oléfines de la formule générale suivante (III) 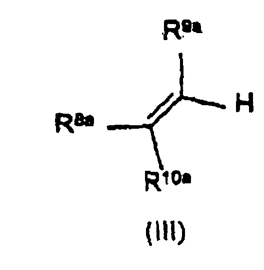 dans laquelle pour les formules du type (II) et (III), R¹ à R³ et R^{1a} à R^{10a} ont la signification indiquée précédemment pour les formules du type (I), et
X signifie de l'iode, du brome, du chlore, OSO₂F₃, OSO₂phényle, OSO₂ tolyl, OSO₂(alkyle en C₁-C₈),
**caractérisé en ce qu'**
on met en oeuvre comme catalyseur un mélange d'un complexe du palladium (O) ou d'un sel de palladium (II) avec des ligands phosphite de formule générale (IVa) ou/et (IVb) 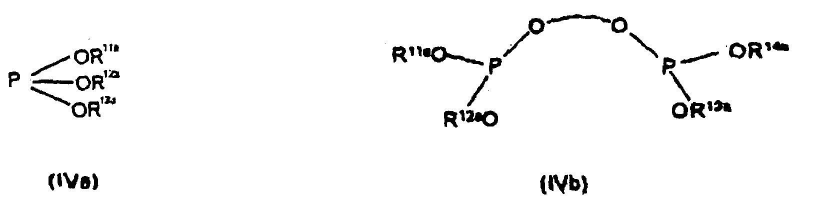 dans lesquelles les restes indiqués R^{11a} à R^{14a} sont identiques ou différents, indépendamment les uns des autres, peuvent signifier un (alkyle en C₁₋C₈), et/ou un groupe alkyle C₁₋C₈, substitué dont les substituants peuvent être de l'hydrogène, un O-alkyle en C₁₋C₈, OCO(alkyle en C₁₋C₈), O-Phényl, Phenyl, Fluor, Chlore OH, NO₂, CN, COOH, CHO, SO₃H, SO₂-Alkyl-(C₁₋C₈), SO-Alkyl-(C₁₋C₈), NH₂, NH-Alkyl-(C₁₋C₈), N-Alkyl₂-(C₁₋C₈), NHCO-Alkyl-(C₁₋C₄), CF₃, COO-Alkyl-(C₁₋C₈), CONH₂, CO-Alkyl₂-(C₁-C₈), NHCOH, NHCOO-Alkyl-(C₁₋C₄), CO-Phenyl, COO-Phenyl, CHCH-CO₂-Alkyl-(C₁₋C₈), CHCHCO₂H, PO-Phenyl₂ ou POAkyl₂-C₁₋C₈)
ou les restes indiqués R^{11a} à R^{14a} signifient un composé aromatique Ar' formule dans laquelle Ar' représente un reste phényle substitué, naphtyle, anthryle, phénanthryle, biphényle et/ou un composé hétéro-aromatique à cinq, six ou sept maillons dans le cycle, pour lesquels ces composés aromatiques peuvent contenir jusqu'à 10 substituants qui représentent un (alkyle en C₁-C₈) OAr', Ar', Fluor, Chlore, OH, NO₂, CN, CO₂H, CHO, SO₃H, NH₂, NH-Alkyl-(C₁₋C₁₂), N-Alkyl₂-(C₁-C₁₂), NHCO-Alkyl-(C₁-C₁₂), CO-Alkyl-(C₁₋C₁₂), NHCHO, COAr', CO₂AR', CF₃, CONH₂, CHCHCO₂H, POAR'₂, PO-Alkyl₂-(C₁₋C₁₂), SiAlkyl₃-(C₁₋C₁₂), O-Alkyl-(C₁-C₈), OCO-Alkyl-(C₁₋C₈), O-Phenyl, Phenyl, COO-Alkyl-(C₁₋C₈), NHCOO-Alkyl-(C₁₋C₄), CO-Phenyl, COO-Phenyl, CHCH-CO₂-Alkyl-(C₁₋C₈), PO-Phenyl₂, PO₃H₂, SO₃-Alkyl-(C₁₋C₄), SO₂-Alkyl-(C₁₋C₄) ou SO-Alkyl-(C₁₋C₄), dans lesquels la formule (IVb) représente un ligand phosphite chélatant, avec un pont carboné en C₂ à C₄, le pont carbone du ligand phosphite entre les deux atomes de carbone pouvant posséder jusqu'à 4 substituants qui sont identiques ou différents et représentent en particulier de l'hydrogène, un (alkyle en C₁₋ C₄), un O-alkyle en C₁₋ C₄, un OH, un aryle ou/et un phényle, dans lesquels aryle a la signification citée ci-dessus.

2. Procédé selon la revendication 1, de préparation de composés de formule (Id)
**caractérisé en ce que**
comme composés hétéro-halogéno-aromatiques Ar X de formule (IId) on met en oeuvre des composés hétéro-halogéno-aromatiques substitués ayant jusqu'à quatre hétéro-atomes comme N, O et/ou S, dans le cycle, de préférence des pyridines substituées, les puprimidines, des oxazoles, des imidazoles, des pyrazines, les quinolines, des indoles, des furanes, des benzofuranes, et/ou des thiophènes, pour lesquels les substituants peuvent être identiques ou différents, indépendamment les uns des autres, de l'hydrogène, un alkyle en C₁₋ C₈, un O-aryle, un aryle, un fluor, Chlore, OH, NO₂, CN, CO₂H, CHO, SO₃H, NH₂, NHAlkyl-(C₁₋C₁₂), NAlkyl-(C₁₋C₁₂), NHCHO-Alkyl-(C₁₋C₁₂), COAlkyl-(C₁₋C₁₂), NHCHO, COAryl, COAryl₂, CF₃, CONH₂, POAryl₂, PO-Alkyl₂-(C₁₋C₁₂), SiAlkyl₃(C₁₋C₁₂), O-Alkyl-(C₁₋C₈), OCO-Alkyl(C₁₋C₈), O-Phenyl, Phenyl, COO-Alkyl-(C₁₋C₈), NHCOO-Alkyl-(C₁-C₄), CO-Phenyl, COO-Phenyl, CHCH-CO₂-Alkyl(C₁-C₈), PO-Phenyl₂, PO₃H₂, SO₃-Alkyl-(C₁-C₄), SO₂-Alkyl-(C₁-C₄) ou SO-Alkyl-(C₁-C₄),
dans lesquels aryle a la signification citée ci-dessus et R^{8a} à R^{10a} qui sont identiques ou différents, indépendamment les uns des autres signifient de l'hydrogène, CN, CO₂H, phényle, CO₂,-alkyle en C₁₋C₈, CONH₂, F, O-(alkyle en C₁₋C₄), (alkyle en C₁₋C₈), NH-alkyle en C₁₋C₄.

3. Procédé selon la revendication 1 ou la revendication 2,
**caractérisé en ce que**
les oléfines sont préparées dans lesquels, dans la formule (Ia), R¹ à R³ représentent de l'hydrogène, un alkyle en C₁₋C₄, ou/et phényle.

4. Procédé de préparation des composés des formules (Ia), (Ib), (lc), ou/et (Id), selon une des revendications 1 à 3,
**caractérisé en ce que**
R¹ à R³ et R^{1a} à R^{7a}, indépendamment l'un de l'autre, signifient de l'hydrogène, un alkyle en C₁₋C₈, O-Alkyl-(C₁-C₈), OCO-Alkyl-(C₁₋C₈), Phenyl, Aryl, Fluor, Chlore, NO₂, CN, COOH, CHO, SO₂-Alkyl-(C₁₋C₄), NH-(C₁₋C₈)Alkyl, N[(C₁-C₈)Alkyl)]₂, COO-(C₁-C₈)-Alkyl, CONH₂, CO-(C₁-C₈)-Alkyl, CO-Phenyl ou/et PO(Phenyl)₂,
pour lesquels aryle a la signification définie ci-dessus et
R⁸ à R^{10a}, indépendamment l'un de l'autre, signifient de l'hydrogène, CN, CO₂H, CO₂-alkyle en C₁-C₈, CONH₂, F, O-Alkyl-(C₁₋C₄), Alkyl-(C₁₋C₈), NH-Alkyl-(C₁₋C₄), CONH-Alkyl-(C₁₋C₈), CON[alkyle-(C₁₋C₈)]₂, Phényle ou/et Aryle
Aryle ayant les significations fournies ci-dessus.

5. Procédé selon l'une des revendications 1 à 4,
**caractérisé en ce que**
comme solvant on utilise des solvants organiques inertes ou/et de l'eau, de préférence des solvants aprotiques dipolaires, des éthers aliphatiques, des hydrocarbones aromatiques ou aliphatiques, des alcools et de l'eau, ainsi que leurs mélanges, en particulier des dialkylsulfoxides, des N, N-dialkylamides d'acide carboxylique aliphatique ou des lactames alkylés, spécialement le dimétylsulfoxyde, le N, N-diméthylacétamide, le N,N-diméthylformamide et la N-méthylpyrolidone

6. Procédé selon l'une des revendications précédentes,
**caractérisé en ce que**
la réaction se déroule à des températures allant de 20 à 200° C, de préférence de 60 à 180° C d'une manière particulièrement préférée de 100 à 160° C, la pression pouvant s'élever jusqu'à 100 bars.

7. Procédé selon l'une des revendications précédentes,
**caractérisé en ce que**
HX qui est clivé au cours de la réaction, est capté par addition d'une base, en particulier avec des alkylamines, des dialkylamines, des trialkylamines qui sont, de préférence, alicycliques ou/et à chaine ouverte, ou/et avec des sels de métal alcalin et/ou de métal alcalinoterreux d'acide aliphatique ou/et aromatique, en particulier avec des acétates, des propionates, des benzoates ou des carbonates correspondants, des hydrogéno-carbonates, des phosphates, des hydrogénophosphates, ou/et des hydroxydes, de préférence de lithium, de sodium, de potassium, de calcium, de magnésium et de césium.

8. Procédé selon l'une des revendications précédentes.
**caractérisé en ce que**
les catalyseurs au palladium (IV) mis en oeuvre sont produits in situ à partir de sels de palladium (II) et des ligands phosphites correspondants.

9. Procédé selon l'une des revendications précédentes,
**caractérisé en ce que**
les catalyseurs au palladium (IV) mis en oeuvre sont utilisés directement sous forme de complexes palladium (O) phosphite.

10. Procédé selon l'une des revendications précédentes,
**caractérisé en ce que**
comme stades précurseurs, on met en oeuvre de l'acétate de palladium (II), du chlorure de palladium (II), du bromure de palladium (II), du tétrachloropalladate de lithium, d'acétylacétonatc de palladium (II), du complexe de dibenzylideneacétone-palladium (O), du palladium (O) tétrakistriphénylphosphane, du palladium (O) bis-tri O-tolylphosphane, du propionate de palladium (II), du chlorure de palladium (II) bisacétonitrile le chlorure de palladium (II) bistriphénylphosphane, le complexe palladium (O) tricyclohexyl-phosphane diallyléther, le palladium (O) bis tricyclohexyl-phosphane et le palladium (II) chlorure bis benzénitrie ou/et d'autres complexes du palladium (O) et du palladium (II).

11. Procédé selon l'une des revendications précédentes,
**caractérisé en ce qu'**
on met en oeuvre un ligand phosphite complexant de formule (IVb) dont le front carboné C₂ à C₄ possède jusqu'à quatre substituants identiques ou différents, en particulier de l'hydrogène, un (allyle en C₁ à C₄), un O-(alkyle en C₁ à C₄), un OH, un aryle ou/et un phényle, aryle ayant la signification citée ci-desus.

12. Procédé selon l'une des revendications précédentes,
**caractérisé en ce qu'**
on met en oeuvre le catalyseur dans un rapport palladium : ligand compris entre 1 :2 et 1 :1000, de préférence entre 1 :5 et 1 :200.

13. Procédé selon l'une des revendications précédentes,
**caractérisé en ce qu'**
on ajoute un co-catalyseur au catalyseur palladium-phosphite, de préférence un sel d'un halogène, en particulier un halogénure des éléments alcalins ou/et des éléments alcalino-terreux, un halogénure d'ammonium, ou/et un halogénure de phosphonium, de préférence un bromure ou/et un chlorure, avant tout LiBr, Lill, NaBr, KBr, C₅ Br Bu₄ NCl, Bu₄ NBr, Bz Me₃ NBr, le bromure de tétraphénylphosphonium, le bromure de trioctylméthylammonium.

14. Procédé selon l'une des revendications précédentes,
**caractérisé en ce qu'**
on ajoute le co-catalyseur en une quantité allant de 0,01% molaire à 100 % molaire, de préférence de 0,1 à 50 % molaire.

15. Procédé selon l'une des revendications précédentes,
**caractérisé en ce qu'**
on utilise des teneurs en catalyseurs de ≤ 2 % molaire, de préférence de ≤ 1 % molaire et d'une manière particulièrement préférée de ≤ 0,2 % molaire.
